# EUROPEAN PATENT SPECIFICATION

(11) **EP 4 260 068 B1**
(45) Date of publication and mention of the grant of the patent: **05.03.2025**
(21) Application number: 21847761.0
(22) Date of filing: 14.12.2021
(51) Int. Cl.: G01N 33/574, G01N 33/50, G01N 33/68

(54) **BIOMARKERS FOR DETECTING CANCER**
BIOMARKER ZUM NACHWEIS VON KREBS
BIOMARQUEURS DE DÉTECTION DE CANCER

(30) Priority: 14.12.2020 GB 202019682
(43) Date of publication of application: 18.10.2023
(73) Proprietor: University of Cape Town, 7700 Cape Town (ZA)
(72) Inventor: LEANER, Virna Drucille, 7700 Rondebosch, Cape Town (ZA); VAN DER WATT, Pauline Janet, 7700 Rondebosch, Cape Town (ZA)
(74) Representative: Barker Brettell LLP
(86) International application number: PCT/IB2021/061690
(87) International publication number: WO 2022/130197

(56) References cited:
- WO-A2-2005/005601
- LI X F ET AL: "IPO5 promotes malignant progression of esophageal cancer through activating MMP7", EUROPEAN REVIEW FOR MEDICAL AND PHARMACOLOGICAL SCIENCES, vol. 24, 1 April 2020 (2020-04-01), pages 4246 - 4254, XP055889937
- WATT PAULINE J. ET AL: "Nuclear transport proteins are secreted by cancer cells and identified as potential novel cancer biomarkers", INTERNATIONAL JOURNAL OF CANCER, vol. 150, no. 2, 30 September 2021 (2021-09-30), US, pages 347 - 361, XP055911199, ISSN: 0020-7136, DOI: 10.1002/ijc.33832

## Description

### FIELD OF THE INVENTION

The invention relates to a method for diagnosing cancer in a subject, the method comprising detecting levels of specific biomarkers in a biological sample from the subject.

### BACKGROUND OF THE INVENTION

Cancer is a leading cause of death globally, accounting for an estimated 9.6 million deaths, or one in six deaths, in 2018. Survival rates of many types of cancers are significantly improved if the cancer is detected early, but large numbers of cancer patients globally do not have access to timely quality diagnosis and treatment. Screening or diagnostic tests that can be performed include physical examination, imaging tests (e.g. computerized tomography (CT) scan, bone scan, magnetic resonance imaging (MRI), positron emission tomography (PET) scan, ultrasound and X-ray) and biopsy. The shortcomings of these screening and diagnostic tests include their low sensitivity, high cost, time-consuming procedures, invasiveness, and risk of bodily exposure to radioactive substances, as well as their requirement for the services of an expert. A few urine and blood tests, which are non-invasive, inexpensive and can provide a quick result, are also known for a limited number of cancers. However, blood tests for cancer are usually based on the detection of tumor markers and will therefore not be detected until a tumor has been established. With the exception of blood cancers, blood tests also often can't distinguish cancer and some other noncancerous conditions, and the use of some tumor-based tests is controversial.

There is therefore still a need for a non-invasive, sensitive, inexpensive and rapid test for cancer.

Li et al., 2020 (Li XF et al., European Review for Medical and Pharmaceutical Sciences, 2020, vol. 24, pages 4246-4254) discloses a method of diagnosing cancer in a subject, by testing a tissue sample from said subject for the presence of Ipo5.

### SUMMARY OF THE INVENTION

According to a first embodiment of the invention, there is provided a method of diagnosing cancer in a subject, the method comprising the step of testing a blood sample from a subject for the presence of Ipo5 (Importin 5) at least one other biomarker, wherein the at least one other biomarker is selected from the group consisting of Ran (Ras-related nuclear protein) and Kpnβ1 (Karyopherin beta 1).

The method may comprise testing the sample for the presence of at least Ipo5 and Ran. The method may comprise testing the sample for the presence of at least Ipo5 and Kpnβ1. The method may comprise testing the sample for the presence of at least Ipo5, Ran and Kpnβ1.

The method may comprise additionally testing the sample for at least one other biomarker selected from the group consisting of Kpnα2, CRM1, CAS and Transportin 1.

The method may comprise testing the sample for the presence of at least 5 biomarkers selected from the group consisting of: Ipo5, Ran, Kpnβ1, Kpnα2, CRM1, CAS and Transportin 1.

The method may comprise testing the sample for the presence of at least 6 biomarkers selected from the group consisting of Ipo5, Ran, Kpnβ1, Kpnα2, CRM1, CAS and Transportin 1.

The method may comprise testing the sample for the presence of the following biomarkers: Ipo5, Ran, Kpnβ1, Kpnα2, CRM1, CAS and Transportin 1.

The cancer may be cervical cancer, oesophageal cancer, breast cancer or liver cancer, or the method may diagnose at least cervical, oesophageal, liver and breast cancer, if any of these are present, in the same test.

The method may diagnose cancer non-specifically.

The sample is a blood sample, such as a serum sample.

Detection of the biomarkers in the sample or a measured signal which equates to a level of biomarker in the sample which is higher than a threshhold level of the same biomarker may be an indicator of cancer.

According to a second embodiment of the invention, there is provided a point-of-care device for diagnosing cancer according to the method described above, the device comprising:
a means for receiving a blood sample from a subject suspected of having cancer:
capture agents for binding Ipo5 and at least one other biomarker, wherein the at least one other biomarker is selected from the group consisting of Ran and Kpnβ1; and
at least one indicator which indicates when the capture agents bind to the biomarkers. The capture agents may be selected from the group consisting of antibodies, affibodies, ankyrin repeat proteins, armadillo repeat proteins, nucleic acid aptamers, peptides, carbohydrate ligands, synthetic ligands and synthetic polymers. Preferably, the capture agents are antibodies.

The indicator may indicate binding of the capture agent to the biomarker by electrical, electronic, acoustic, optical or mechanical methods.

The device may further include measuring means for measuring the levels of the detected biomarkers.

The device may further include amplifying means for increasing the sensitivity of the detection of the biomarkers.

The device may be a hand-held point-of-care device.

According to a third embodiment of the invention, there is provided a kit for diagnosing cancer according to the method described above, the kit comprising the following:
capture agents for binding Ipo5 and at least one other biomarker, wherein the at least one other biomarker is selected from the group consisting of Ran and Kpnβ1;
means for obtaining or receiving a sample from a subject;
a point-of-care device for diagnosing cancer according to the second embodiment; and
instructions, in electronic or paper form, for performing the method as described above.

According to a further embodiment of the invention, there is provided a computer implemented method for diagnosing cancer in a subject according to the method described above, the computer performing steps comprising:
receiving inputted subject data comprising values for levels of at least five biomarkers selected from the group consisting of Ipo 5 and at least one other biomarker, wherein the at least one other biomarker is selected from the group consisting of Ran and Kpnβ1;
comparing these values with predetermined values for the biomarkers;
determining whether the subject has cancer; and
displaying information regarding the diagnosis of the subject.

### BRIEF DESCRIPTION OF THE FIGURES

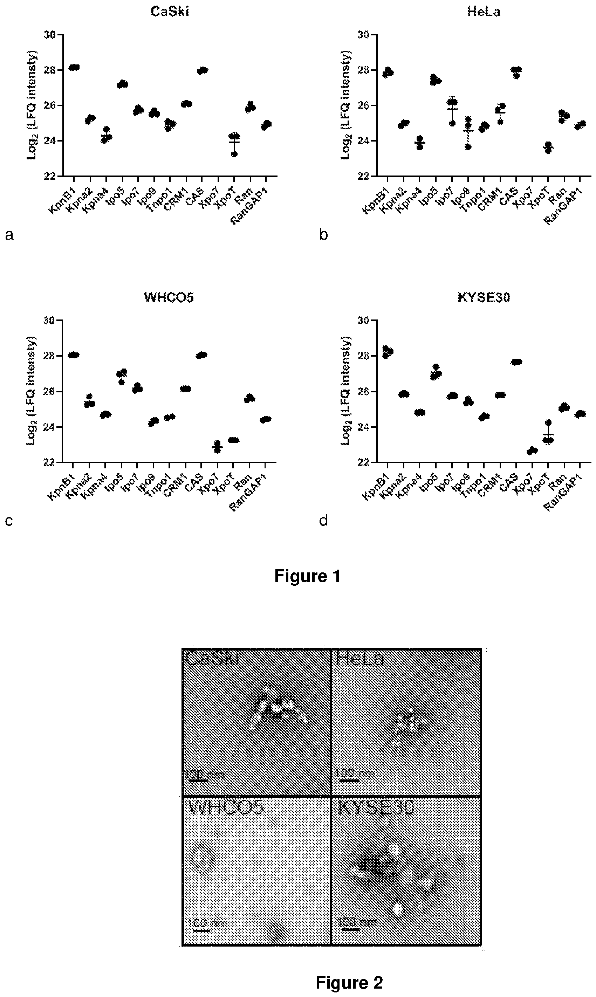

Figure 1: Identification of nuclear transport proteins in the secretome and exosomes isolated from cervical cancer and oesophageal cancer cell lines. LFQ intensity plots showing the nuclear transport proteins present in CaSki (a), HeLa (b), WHCO5 (c) and KYSE30 (d) cancer cell secretomes. The mean ± SD is shown.
Figure 2: Identification of nuclear transport proteins in the secretome and exosomes isolated from cervical cancer and oesophageal cancer cell lines. TEM images of extracellular vesicles isolated by differential ultracentrifugation from CaSki (a), HeLa (b), WHCO5 (c) and KYSE30 (d) cells. Scale bar = 100 nm.

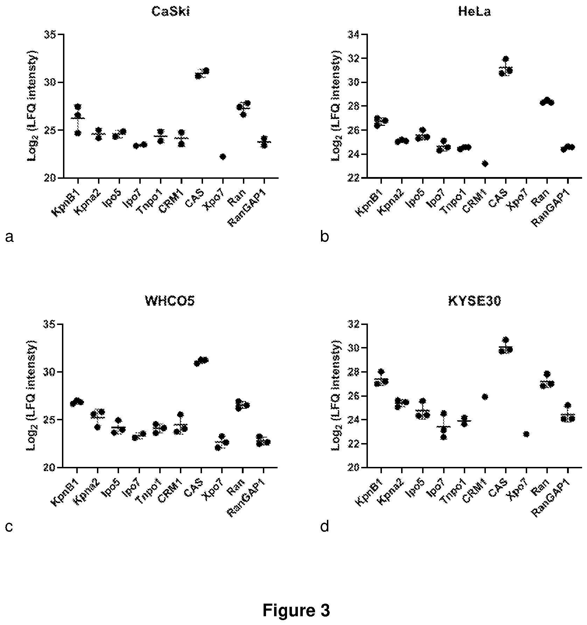

Figure 3: Identification of nuclear transport proteins in the secretome and exosomes isolated from cervical cancer and oesophageal cancer cell lines. LFQ intensity plots showing the nuclear transport proteins present in CaSki (a), HeLa (b), WHCO5 (c) and KYSE30 (d) exosome sets (where experimental replicates are represented as individual dots).

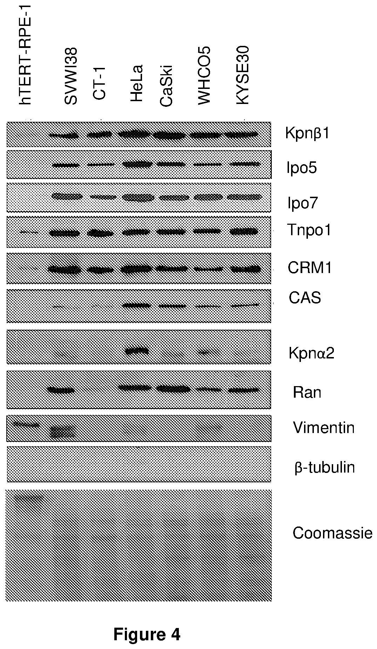

Figure 4: Secretion of nuclear transport proteins by cultured normal, transformed, cervical cancer and oesophageal cancer cell lines. Western blot analysis of Kpnβ1, Ipo5, Ipo7, Tnpo1, CRM1, CAS, Kpnα2 and Ran secreted by the normal immortalized epithelial cell line (hTERT-RPE1), transformed cell lines (SVWI38 and CT-1), cervical cancer cell lines (HeLa and CaSki) and oesophageal cancer cell lines (WHCO5 and KYSE30). Coomassie staining was performed to show the protein profile along each lane. β-tubulin was used to monitor cytosolic contamination of the cell secretome. Vimentin was used to show that not all proteins are secreted at elevated levels by cancer cells. Results shown are representative of experiments performed in quadruplicate.

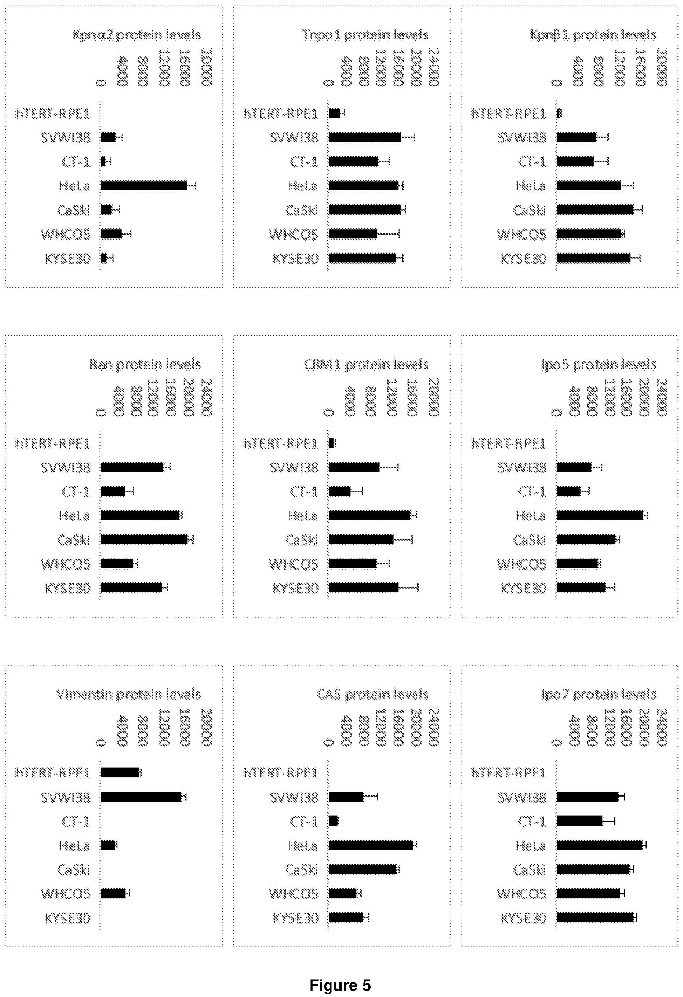

Figure 5: Secretion of nuclear transport proteins by cultured normal, transformed, cervical cancer and oesophageal cancer cell lines. Densitometry quantification of secreted nuclear transport proteins by cultured normal, transformed, cervical cancer and oesophageal cancer cell lines. Image J was used to quantify band intensities. Results shown represent the mean ± SEM of experiments performed in quadruplicate (*indicates significance compared to hTERT-RPE-1; p< 0.05).

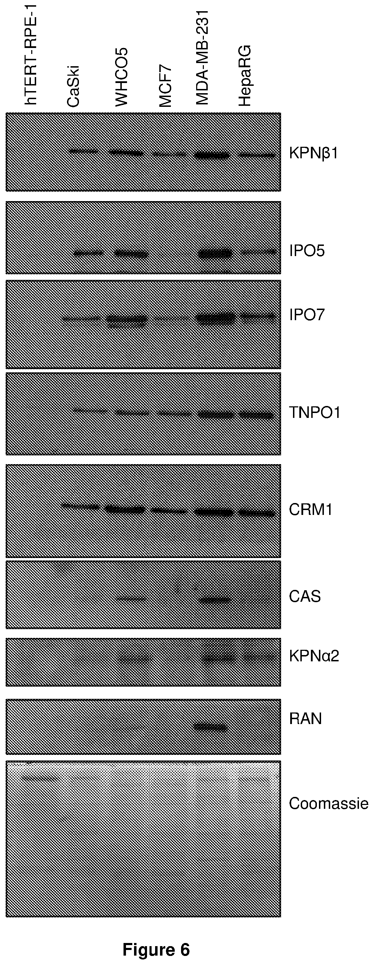

Figure 6: Secretion of nuclear transport proteins by different cancer types. Western blot analysis of KPNβ1, IPO5, IPO7, TNPO1, CRM1, CAS, KPNα2 and RAN secreted by normal immortalised epithelial cells (hTERT-RPE-1), cervical cancer cells (CaSki), oesophageal cancer cells (WHCO5), breast cancer cells (MCF-7 and MDA-MB-231) and liver cancer cells (HepaRG). Coomassie staining was performed to show the protein profile along each lane.

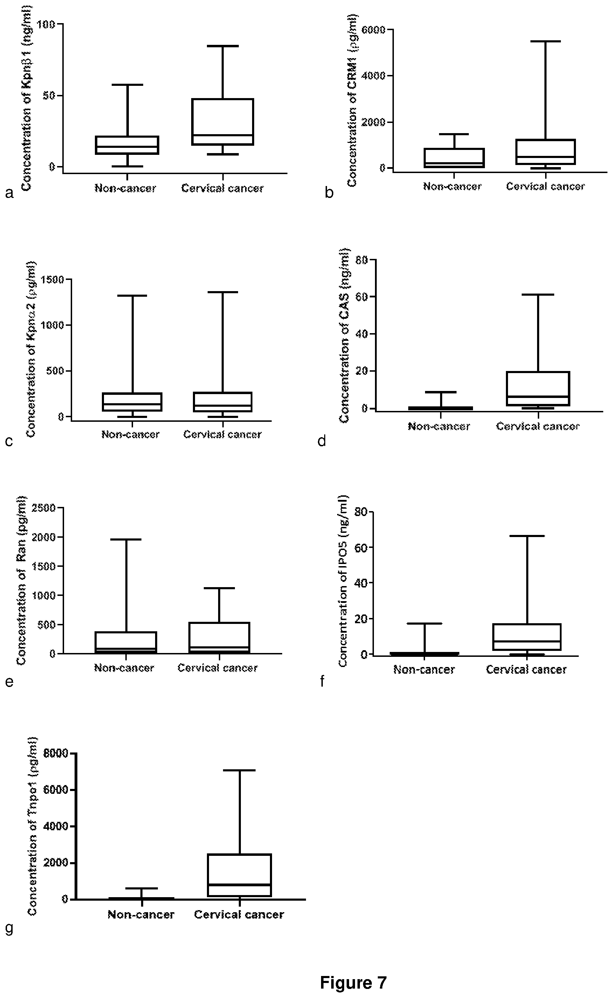

Figure 7: Serum level of Kpnβ1, CRM1, Kpnα2, CAS, Ran, Ipo5 and Tnpo1 in non-cancer subjects and cervical cancer patients. ELISA assays were used to determine the concentration of serum Kpnβ1 (a), CRM1 (b), Kpnα2 (c), CAS (d), Ran (e), Ipo5 (f) and Tnpo1 (g) in non-cancer controls and cervical cancer cases.

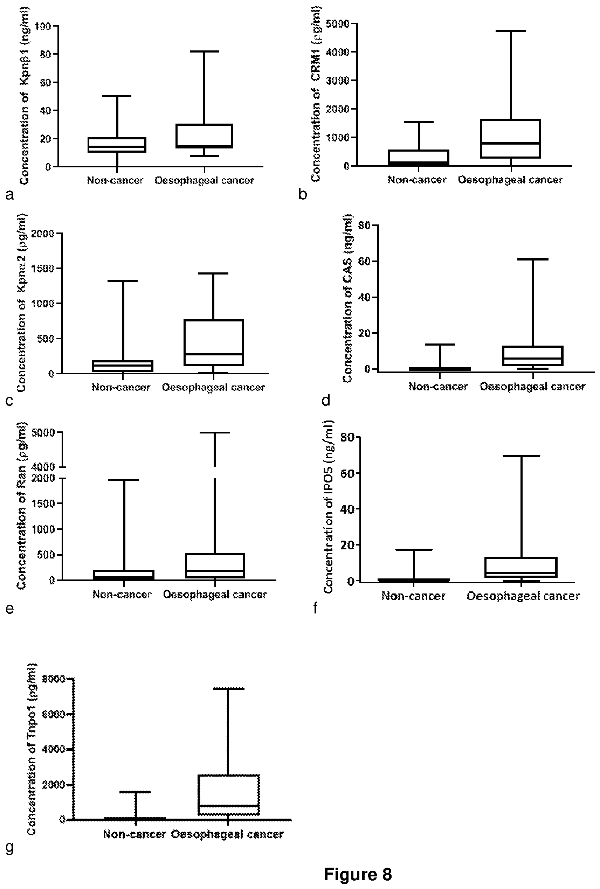

Figure 8: Serum level of Kpnβ1, CRM1, Kpnα2, CAS, Ran, Ipo5 and Tnpo1 in non-cancer subjects and oesophageal cancer patients. ELISA assays were used to determine the concentration of serum Kpnβ1 (a), CRM1 (b), Kpnα2 (c), CAS (d), Ran (e), Ipo5 (f) and Tnpo1 (g) in non-cancer controls and oesophageal cancer cases. Data are represented as box and whisker plots. The non-parametric Mann-Whitney U-Test was used to compare non-cancer subjects and cancer cases (*P<0.05 and ***P*<0.0005).

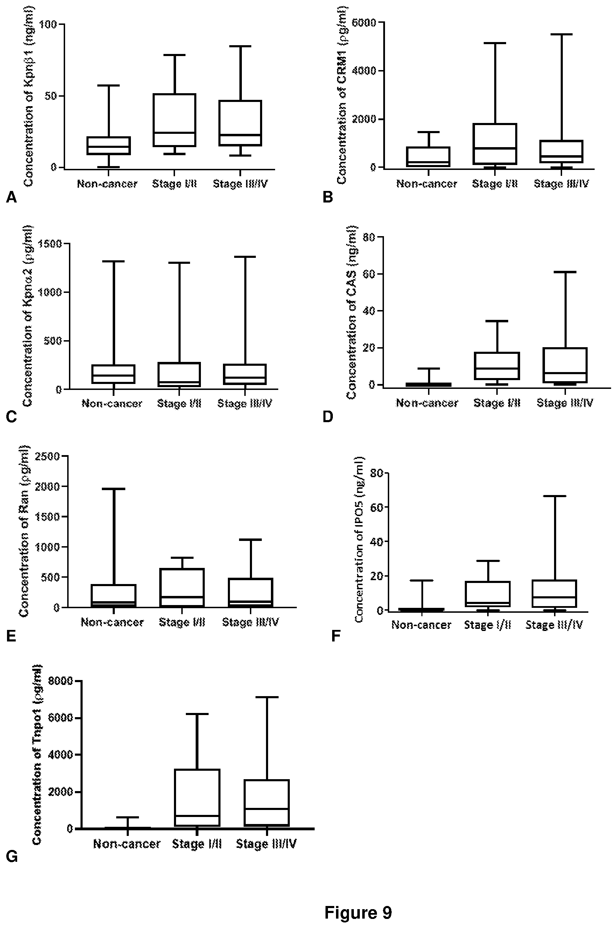

Figure 9: Serum level of Kpnβ1, CRM1, Kpnα2, CAS, Ran, Ipo5 and Tnpo1 in non-cancer subjects, early-stage and late-stage cervical cancer patients. ELISA assays were used to determine the concentration of serum Kpnβ1 (A), CRM1 (B), Kpnα2 (C), CAS (D), Ran (E), Ipo5 (F) and Tnpo1 (G) in non-cancer controls, early-stage and late-stage cervical cancer cases (**P*<0.05 and ***P*<0.0005).

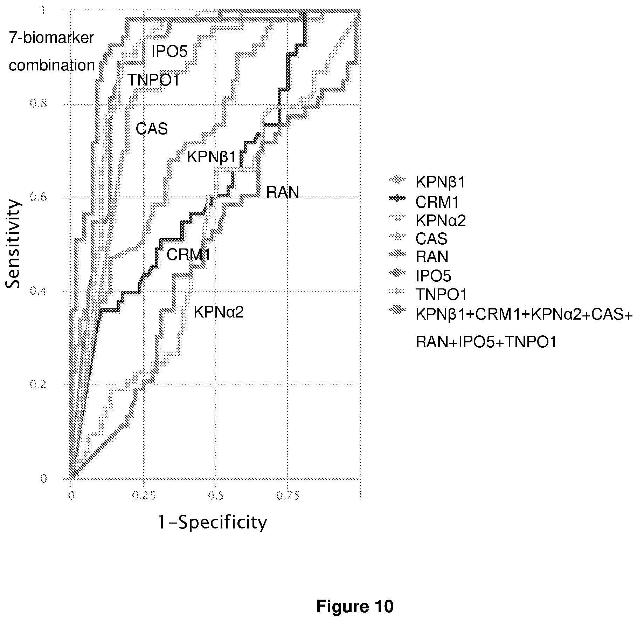

Figure 10: ROC curves showing the ability of Kpnβ1, CRM1, Kpnα2, CAS, Ran, Ipo5, Tnpo1 and their combination to discriminate cervical cancer cases from non-cancer controls.

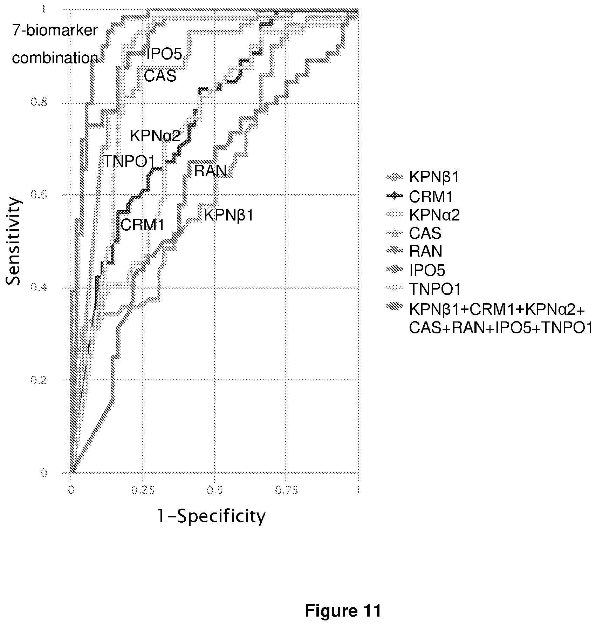

Figure 11: ROC curve showing the ability of Kpnβ1, CRM1, Kpnα2, CAS, Ran, Ipo5, Tnpo1 and their combination to discriminate oesophageal cancer cases from non-cancer controls.

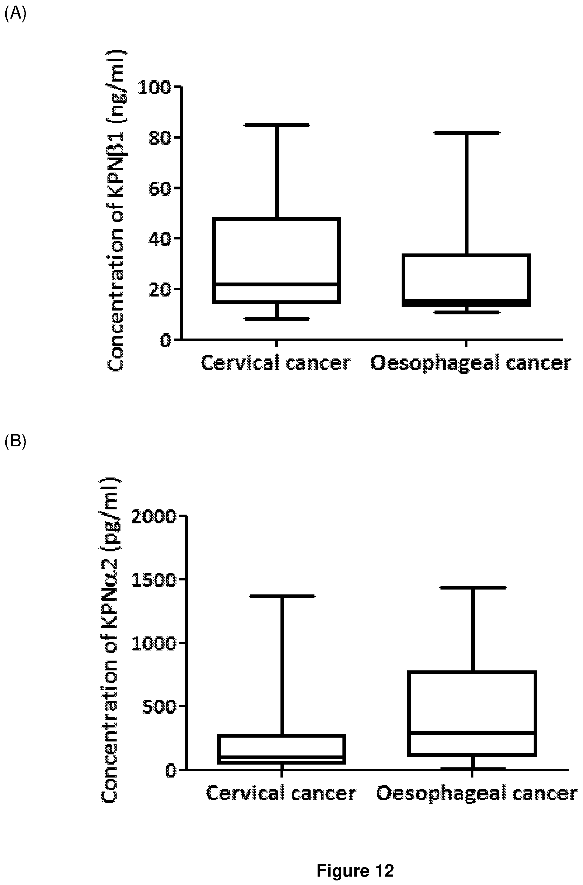

Figure 12: Serum level of KPNβ1 and KPNα2 in cervical cancer and oesophageal cancer patients. ELISA assays were used to determine the concentration of serum KPNβ1 (A) and KPNα2 (B) in cervical cancer compared to oesophageal cancer cases. The non-parametric Mann-Whitney U-Test was used to compare cervical cancer and oesophageal cancer cases (**P*<*0.05* and ***P*<*0.0005*)*.* (C) ROC curve showing the ability of KPNβ1 and KPNα2 (in combination) to discriminate cervical cancer cases from oesophageal cancer cases.

### LIST OF ABBREVIATIONS

- Kpnβ: Karyopherin beta
- Kpnα: Karyopherin alpha
- Ran: Ras-related nuclear protein
- CAS: Cellular apoptosis-susceptibility protein
- Ipo5: Importin 5
- Tnpo1: Transportin 1
- ROC: Receiver operating characteristics
- AUC: Area under the receiver operating characteristics curve

### DETAILED DESCRIPTION OF THE INVENTION

A method, device, kit and computer-implemented method for diagnosing (and optionally also treating) cancer are described herein.

The method may comprise testing the sample for the presence of at least 2 biomarkers, at least 3 biomarkers, at least 4 biomarkers, at least 5 biomarkers, at least 6 biomarkers or at least 7 biomarkers selected from the group consisting of Ipo5, Ran, Kpnβ1, Kpnα2, CRM1, CAS and Transportin 1.

At least one of the biomarkers is Ipo5. At least one of the other biomarkers is either of Ran or Kpnβ1.

The method can further comprise testing the sample for a third biomarker. In one embodiment, the third biomarker is CRM1. In an alternative embodiment, the third biomarker is Kpnα2. In an alternative embodiment, the third biomarker is CAS. In an alternative embodiment, the third biomarker is Ran. In an alternative embodiment, the third biomarker is Ipo5. In an alternative embodiment, the third biomarker is Transportin 1. In an alternative embodiment, the third biomarker is Kpnβ1.

The method can further comprise testing the sample for a fourth biomarker. In one embodiment, the fourth biomarker is CRM1. In an alternative embodiment, the fourth biomarker is Kpnα2. In an alternative embodiment, the fourth biomarker is CAS. In an alternative embodiment, the fourth biomarker is Ran. In an alternative embodiment, the fourth biomarker is Ipo5. In an alternative embodiment, the fourth biomarker is Transportin 1. In an alternative embodiment, the fourth biomarker is Kpnβ1.

The method can further comprise testing the sample for a fifth biomarker. In one embodiment, the fifth biomarker is CRM1. In an alternative embodiment, the fifth biomarker is Kpnα2. In an alternative embodiment, the fifth biomarker is CAS. In an alternative embodiment, the fifth biomarker is Ran. In an alternative embodiment, the fifth biomarker is Ipo5. In an alternative embodiment, the fifth biomarker is Transportin 1. In an alternative embodiment, the fifth biomarker is Kpnβ1.

The method can further comprise testing the sample for a sixth biomarker. In one embodiment, the sixth biomarker is CRM1. In an alternative embodiment, the sixth biomarker is Kpnα2. In an alternative embodiment, the sixth biomarker is CAS. In an alternative embodiment, the sixth biomarker is Ran. In an alternative embodiment, the sixth biomarker is Ipo5. In an alternative embodiment, the sixth biomarker is Transportin 1. In an alternative embodiment, the sixth biomarker is Kpnβ1.

The method can further comprise testing the sample for a seventh biomarker. In one embodiment, the seventh biomarker is CRM1. In an alternative embodiment, the seventh biomarker is Kpnα2. In an alternative embodiment, the seventh biomarker is CAS. In an alternative embodiment, the seventh biomarker is Ran. In an alternative embodiment, the seventh biomarker is Ipo5. In an alternative embodiment, the seventh biomarker is Transportin 1. In an alternative embodiment, the seventh biomarker is Kpnβ1.

More particularly, the method can comprise testing the sample for the following combinations:
i) Ipo5 and Ran;
ii)
Ipo5 and Kpnβ1;
iv) Ipo5 and Ran, and at least one of Kpnβ1, CRM1, Kpnα2, CAS and Transportin 1;
v) Ipo5 and Ran, and at least one of Kpnβ1 and Kpnα2;
vi) Ipo5, Ran and Kpnβ1;
vii) Ipo5, Ran and Kpnα2;
viii) Ipo5 and Ran, and at least two of Kpnβ1, CRM1, Kpnα2, CAS and Transportin 1;
ix) Ipo5 and Ran, and at least three of Kpnβ1, CRM1, Kpnα2, CAS and Transportin 1;
x) Ipo5 and Ran, and at least four of Kpnβ1, CRM1, Kpnα2, CAS and Transportin 1;
xi) Ipo5, Ran, Kpnβ1, CRM1, Kpnα2, CAS and Transportin 1;
xii) Ipo5, Kpnβ1, CRM1, Kpnα2, CAS and Ran;
xiii) Ipo5, Kpnβ1, CRM1, CAS and Transportin 1;
xiv) Ipo5, Kpnβ1, CRM1, Kpnα2, CAS and Ran;
xv) Ipo5, Kpnβ1, CRM1, Kpnα2, CAS, Ran and Transportin 1.

The cancer can be any cancer and is typically a solid tumour such as gynaecological cancers (e.g. cervical cancer), oesophageal cancer, breast cancer, ovarian cancer, gastro-intestinal cancers (e.g. gastric cancer), prostate cancer, pancreatic cancer, lung cancer, liver cancer, colorectal cancer, stomach cancer, neuroblastoma and gliomas. In one embodiment, the method is used to detect cervical cancer; in another embodiment the method is used to detect oesophageal cancer; in another embodiment the method is used to detect breast cancer; in another embodiment the method is used to detect liver cancer. In an alternative embodiment the method is used to detect cancer in general if it is present in the subject. In this embodiment, a positive diagnosis will be non-specific, i.e. it will not indicate the type of cancer that the subject has.

A positive diagnosis for cancer can be made when two, three, four, five, six or seven of the tested biomarkers are detected, or when the levels of the detected biomarkers are higher than a typical level of the same biomarker in subjects without cancer.

Cut-off or threshold values can be determined based on levels of biomarkers which are typically found in patients without cancer, and the levels of the biomarkers detected in the sample can be compared to the cut-off levels when making the determination of whether or not the subject has cancer. In other words, the method will detect whether the biomarkers in the panels are under- or over-expressed relative to a subject who does not have cancer. The method can also be used as an initial screening tool to determine whether a further diagnostic test should be performed to confirm that the patient has cancer (e.g. a biopsy or MRI) or to confirm the type of cancer that the patient has.

The method can be used to detect cancer at an early stage, e.g. when a tumour has not yet been established or when biomarkers found in tumour tissue are not yet detectable.

The sample is a blood sample, which can be whole blood, serum or plasma.

Typically, the sample is a serum sample. The serum sample can be diluted prior to testing, e.g. with a buffer, or can be tested without dilution.

Cancer treatment can be administered to subjects who are identified as having cancer (e.g. chemotherapy and/or radiation), and/or surgery can be performed to remove the cancer. The biomarkers can be detected using commercially available techniques, such as laboratory-based techniques including ELISA, micro-array or multiplex bead array technology.

In one embodiment, the device has a means for receiving the sample from the subject, such as a loading or receiving area onto or into which the sample is placed. Capture agents and indicators are present in the device, and once the sample has been loaded onto or received into the device, the sample is brought into contact with the capture agents, which are allowed to bind to the biomarkers if present. The indicator will signify that binding has occurred. The device may further include amplifying means for increasing the sensitivity of the detection of the biomarkers.

The capture agents can be antibodies, affibodies, ankyrin repeat proteins, armadillo repeat proteins, nucleic acid aptamers, peptides, carbohydrate ligands, synthetic ligands or synthetic polymers. Typically, however, the capture agents are antibodies. The indicator can be a calorimetric, electrical, electrochemical, electronic, chromogenic, optical, fluorescent or a radio-labeled indicator.

A kit can also be provided to enable the method of the invention to be performed. The kit could include one or more of the following:
capture agents, such as antibodies, for binding the intended biomarkers;
a means for obtaining or receiving a blood sample from a subject;
a point-of-care device; and/or
instructions, in electronic or paper form, for performing the method.

The invention further provides a computer implemented method for diagnosing cancer in a subject, the computer performing steps comprising:
a) receiving inputted subject data comprising values for levels of the biomarkers of interest in a blood sample from the subject;
b) comparing these values with predetermined values for the biomarkers;
c) determining whether the subject has cancer; and
d) displaying information regarding the diagnosis of the subject.

The invention will now be described in more detail with reference to the following non-limiting examples.

### Examples:

### Cell lines and cell culture

The human telomerase-immortalized retinal pigmented epithelial 1 (hTERT RPE-1) cell line (RRID: CVCL_4388), SV40-transformed WI38 lung fibroblasts (SVWI38) (RRID: CVCL_2759), human cervical carcinoma cell lines, HeLa (RRID: CVCL_0030) and CaSki (RRID: CVCL_1100), and breast cancer cell lines, MCF-7 (RRID: CVCL_0031) and MDA-MB-231 (RRID: CVCL_0062), were purchased from the American Type Culture Collection (ATCC). The human oesophageal squamous cell carcinoma (WHCO5) cell line was originally established from a South African patient with oesophageal squamous cell carcinoma and acquired from Professor Rob Veale at the University of Witwatersrand while the human oesophageal squamous cell carcinoma (KYSE30) cell line was acquired from DSMZ (RRID: CVCL_1351). HepaRG cells (RRID: CVCL_9720) were a kind gift from Dr Hlumani Ndlovu (University of Cape Town, South Africa) and purchased from Lonza. Cells were maintained in Dulbecco's Modified Eagle's Medium (DMEM) (Gibco^{®}, Life Technologies) supplemented with penicillin, streptomycin and 10% fetal calf serum (FCS) (HyClone Laboratories), whereas hTERT-RPE1 cells were grown in DMEM/F-12 (1:1) nutrient mixture (Gibco^{®}, Life Technologies) supplemented with penicillin, streptomycin, 10% FCS and 0.01 mg/ml hygromycin B (Sigma Aldrich). All cells were maintained in a humidified incubator at 37 °C and in 5% carbon dioxide. Cell lines were authenticated by DNA profiling within the last three years, using the Cell ID system (Promega) and mycoplasma tested by the Hoescht DNA staining method. All experiments were performed with mycoplasma-free cells.

### Protein harvest from serum-free culture media

Cells were grown to 70% confluency, washed twice with serum-free media and incubated in serum-free medium for 24 hours. Conditioned media was centrifuged at 500 × *g* for 15 minutes at 4 °C. The supernatant was transferred into Amicon Ultra-15 centrifugal filter units (Merck Millipore Ltd) for a series of centrifugation steps at 2000 × *g* to concentrate the protein. Protein concentrations were determined using the Bicinchoninic acid (BCA) assay kit (Pierce, Thermo Scientific).

### Isolation of exosomes by ultracentrifugation

9 x 10 cm dishes of respective cells were grown to 80% confluency and serum starved for 24 hr, following which medium was removed and cells were further serum starved for 24 hr. Conditioned media was removed from cells and centrifuged at 380 × *g* for 10 min to remove cells and re-centrifuged at 1500 × *g* for 20 min to remove dead cells. The supernatant was transferred to ultracentrifuge tubes and centrifuged at 9800 × *g* for 30 min at 4°C in a Beckman L-80 ultracentrifuge to remove cell debris. The supernatant was then transferred to clean ultracentrifuge tubes and centrifuged at 100 000 × *g* for 70 min at 4°C to pellet the exosomes and contaminating secreted protein. The supernatant was removed and the pellet re-suspended in 1× PBS. This was then re-centrifuged at 100 000 × *g* for 70 min at 4°C to wash out contaminating secreted protein, the supernatant removed and the pellet re-suspended in RIPA buffer (10 mM Tris-Cl, pH 7.4, 150 mM NaCl, 0.1 % SDS) for mass spectrometry analysis, or re-suspended in 50 µl of 1x PBS for analysis by transmission electron microscopy (TEM).

### Transmission electron microscopy

Preparation for TEM and instrument usage was carried out at the Centre for Imaging & Analysis, University of Cape Town (UCT CIA). Briefly, exosomes suspended in PBS were dried onto a glow discharged carbon coated TEM grid (Agar Scientific), rinsed twice with dH₂0 and negatively stained with 2% aqueous uranyl acetate. The grids were then observed with a FEI Tecnai 20 equipped with a LaB6 emitter and operated at 120 kV. Images were captured using a Gatan Tridiem GIF filter with a 2kX2k CCD camera (Gatan).

### Mass spectrometry

### Filter assisted sample preparation (FASP) method

Three biological replicates per cell line were used, with each sample containing up to 200 µg of protein. Protein samples were resuspended in RIPA buffer and heated for 10 min at 95°C. Samples were denatured using 8 M UA solution (8 M urea in 0.1 M Tris-Cl, pH 8.5) and reduced with 100 mM DTT for 30 min at RT. The samples were concentrated on an Ultracel 30 kDa molecular weight cut off (MWCO) centrifugal filter unit (Amicon Ultra, Merck Millipore Ltd) and the flow-through discarded. Samples were alkylated on-filter with 0.05 M iodoacetamide (IAA) in the dark for 20 min at RT. The filters were then centrifuged at 14 000 × *g* for 10 min and 8 M UA added and the filters centrifuged at 14 000 × *g* for 15 min; this step was repeated twice. 0.05 M ammonium bicarbonate (ABC) buffer was added to the filters, which were then centrifuged at 14 000 × *g* for 10 min at RT; this step was repeated twice. Having confirmed a pH of around 8 for optimal trypsin digestion, samples were incubated on-filter with trypsin (New England Biolabs) in ABC containing 20 mM CaCl₂, at a molar ratio of 1:50 (trypsin:protein concentration). Digestions were performed for 16 hr in a wet chamber at 37°C, after which filters were centrifuged at 14 000 × *g* for 10 min to elute tryptic peptides, followed by the addition of ABC and subsequent re-centrifugation at 14 000 × *g* for 10 min. This step was repeated 3 times. The filtrates were combined and formic acid was added to a final concentration of 0.1% to acidify the sample.

### Desalting of peptides

Following FASP, desalting of peptides was performed to purify and concentrate the peptides using C18 "stage" tips. Stage tips were prepared with Empore Octadecyl solid phase extraction disks (Supelco, Sigma) and activated using 80% acetonitrile (ACN) and 0.1% formic acid. 2% ACN and 0.1% formic acid was added and the stage tip centrifuged at 2000 × *g* for 3 min, three times. 10 µg of trypsin-digested sample was added to the stage tip and centrifuged at 2000 × *g* for 30 sec. 2% ACN and 0.1% formic acid was added and the stage tip centrifuged at 2000 × *g* for 5 min, three times. A glass insert was added into a fresh Eppendorf tube and the stage tip transferred such that elution of the protein would be captured in the glass insert. 60% ACN and 0.1% formic acid was added and the stage tip centrifuged at 2800 × *g* for 5 min at RT, repeated 3 times, eluting onto the glass insert. The glass insert was then air dried for 30 min and stored at -20°C. The pellet was re-suspended in of 2% ACN and 0.1% formic acid to prepare a 200 ng/µl sample for analysis by mass spectrometry.

### Liquid chromatography

Chromatography separation was performed using a home packed 100 µm ID × 20 mm precolumn connected to a 75 µm × 20 cm analytical column packed with C18 Luna beads of 5 µm diameter and 100 Å pore size (Phenomenex 04A-5452). The columns were connected to an Ultimate 3500 RS nano ultra-performance liquid chromatography (UPLC) system (Dionex). Following optimization of ideal loading, 100 ng of secretome peptide samples or 400 ng exosome peptide samples were loaded onto the column with a starting mobile phase of 2% ACN and 0.1% formic acid. Peptides were eluted with the following optimized gradient: 12 min at 2% ACN, increased to 6% ACN in 2 min, to 40% in 118 min, increased to 80% in 5 min and finally kept at 80% for 15 min (total gradient time of 150 min). This was followed by a column wash of 85% ACN for 20 min. The flow rate was 400 nl/min at a temperature of 40°C with a backpressure value during separation of < 350 bar. The column was washed between every run.

### Mass spectrometry data acquisition

The mass spectra of interest for final peptide determination were acquired with an Orbitrap Q Exactive mass spectrometer (Thermofisher), with switching between MS and MS/MS, using an inclusion list of proteins of interest. MS spectra were acquired with a resolution of 70 000, a target value of 3 × 10⁶ ions (maximum integration time of 250 ms) and a scan range of 300 to 1750 *m*/*z.* Peptide fragmentation was performed using higher-energy collision dissociation (HCD) with normalized collision energy set at 28 and intensity threshold for ions selection was 1.3 e4 with charge exclusion of charge number (*z) =* 1 and z> 5. MS/MS spectra were acquired at a resolution of 17 500 with target value of 1 × 10⁵ ions (maximum integration time of 120 ms). The isolation window was set at 2.0 *m*/*z.* An inclusion list of nuclear transport proteins was generated using SkyLine (64-bit) version 3.5.0.9319. (MacCoss Lab Software) (131). 30 nuclear transport proteins were digested *in-silico* into peptides with various charge states and modifications and these were preferentially targeted during the MS run in real time.

### Data analysis

MaxQuant (version 1.5.3.12) was used for raw Xcalibur file processing. Andromeda search engine with the human reference proteome (UniProt Feb 2016, UP000005640), was used to analyse the mass spectra, with a reversed reference proteome as a target decoy database. MS/MS database search was performed using a 20 ppm mass tolerance for precursor ions and 0.5 Da for fragment ions. Cysteine carbamidomethylation was selected as a fixed modification and oxidation of methionine and N-terminal acetylation were selected as variable modifications. Trypsin/P was selected as protease with maximum missed cleavages set at 2. The minimum cut off for peptide length was set at seven amino acids. Results were filtered by a 0.01 false discovery rate at the protein and peptide level. Label free quantification (LFQ) was used to compare peptides across runs. Computations through MaxQuant were performed using facilities provided by the University of Cape Town's ICTS High Performance Computing team.

The resulting text output files were imported into Perseus statistical package software version 1.5.5.3. for further data clean-up, processing, visualization and statistical analysis. This included data clean-up with removal of potential contaminants, reverse hits, peptides only identified by site and then only picking protein groups with at least one unique peptide. Vesiclepedia, a manually curated compendium that contains identities of proteins from all classes of extracellular vesicles (EVs), was used to provide evidence for substantial enrichment of vesicles in the samples. Functional enrichment analyses were performed using FunRich software.

### Western blot analysis

Western blot analysis was performed using rabbit anti-NTF97/Importin beta (Abcam ab45938), rabbit anti-CRM1 (H-300) (Santa Cruz Biotechnology, sc-5595), rabbit anti-KPNα2 (Abcam Ab97580), mouse anti-CAS (Sigma-Aldrich SAB1400055), mouse anti-RAN (Sigma-Aldrich R4777), mouse anti-TNPO1 (Sigma-Aldrich T0825), rabbit anti-IPO5 (Sigma-Aldrich SAB4200179), rabbit anti-IPO7 (H-78) (Santa Cruz Biotechnology sc-134913), mouse anti-GAPDH (0411) (Santa Cruz Biotechnologysc-47724), rabbit anti-β-tubulin (H-235) (Santa Cruz Biotechnologysc-9104) and mouse anti-Vimentin (V9) (Santa Cruz Biotechnologysc-6260) antibodies.

### Blood sample collection

Patients were recruited at Groote Schuur Hospital (Cape Town, South Africa). Ethics approval for blood collection and patient consent were established before the study began (Ethics Reference Number HEC040/2005). Blood from 73 non-cancer subjects, 74 cervical cancer and 73 oesophageal cancer patients were collected into EDTA vacutainer tubes, the tubes inverted several times and incubated at room temperature for 1 hour until coagulation was complete. The tubes were centrifuged at 2000 × *g* for 10 minutes at 4 °C and the supernatant (serum) removed and stored at -20 °C.

### Enzyme-linked immunosorbent assay (ELISA)

ELISA kits for human KPNβ1 (#SEE752Hu), human CRM1/XPO1 (#LS-F9390-1) and human KPNα2 (#SEE748Hu) were purchased from The Cloud-Clone Corp. (Houston, USA). ELISA kits for human CAS/XPO2 (#MBS9316513), human IPO5 (#MBS9311906) and human TNPO1 (#MBS041124) were purchased from MyBiosource, Inc. (San Diego, USA) and for human RAN (#abx382676) from Abbexa Ltd (Cambridge, UK). ELISA kits were used to quantitate KPNβ1, CRM1, KPNα2, CAS, RAN, IPO5 and TNPO1 in patient serum samples according to the manufacturer's instructions, with slight modifications. Serum samples for KPNβ1, CRM1 and KPNα2 analysis were diluted in 0.01M PBS, pH 7.15, while serum samples for CAS, IPO5 and TNPO1 analysis were diluted in sample diluent provided by the manufacturer, and serum samples for RAN analysis were left undiluted. The dilution factors used for quantitating KPNβ1 was 1:5 and for CRM1, KPNα2, CAS, IPO5 and TNPO1 was 1:2.

### Statistical analysis

Microsoft Excel, GraphPad Prism software version 5.01 and CombiROC were used for statistical analyses of Western blot and ELISA data. The unpaired two-tailed Student's t-test was used for statistical analysis of Western blot data and the non-parametric Mann-Whitney *U*-test was used for ELISA data comparisons based on clinical characteristics, with p-values less than 0.05 considered statistically different. Receiver operating characteristics (ROC) curves were constructed by plotting sensitivity (true positive rate) against 1-specificity (false positive rate) by assuming that each value is a possible cut-off point. The area under the receiver operating characteristics curve (AUC) was computed as an index to determine the ability of each biomarker to discriminate between cases and controls. An optimal cut-off value was determined for each candidate biomarker by selecting the cut-off value which corresponded to the highest Youden's index (J)^{42,43}. J is calculated as: J = 1 - (false positive rate + false negative rate); J = 1 - ((1 - specificity) + (1 - sensitivity)); J = 1 - 1 + specificity - 1 + sensitivity; J = sensitivity + specificity - 1

### Results

### Mass spectrometry identification of nuclear transport proteins in the secretome and exosome fraction of cultured cervical cancer and oesophageal cancer cell lines

Mass spectrometry was used to analyse the secretome of two cervical cancer (HeLa and CaSki) and two oesophageal cancer (WHCO5 and KYSE30) cell lines. An inclusion list of 30 nuclear transport proteins was generated and incorporated into the mass spectrometry method (Table 1). Briefly, serum-free conditioned media were concentrated from cells, prepared for mass spectrometry analysis using the filter assisted sample preparation (FASP) method, subjected to trypsin digestion, and analysed with an Orbitrap Q Exactive mass spectrometer. Experiments were performed in triplicate for each cell line, and strong Pearson correlation coefficients were obtained when triplicate samples were compared to each other (on average > 0.9, data not shown). The total number of protein groups in all three replicates were 740, 466, 886 and 845, for CaSki, HeLa, WHCO5 and KYSE30, respectively. A 48-60 % similarity in overall protein identities was identified amongst cell lines. Interestingly, amongst the identified proteins in the secretome of each of the cervical and oesophageal cancer cells, 13 were members of the nuclear transport protein family, including KPNβ1, KPNα2, KPNα4, IPO5, IPO7, IPO9, TNPO1, CRM1, CAS, XPO7, XPOT, RAN and RANGAP1 (Figure 1).

**Table 1: List of nuclear transport proteins in an inclusion list generated using SkyLine**

| **Importins** | |
|---|---|
| Kpnβ1/Ipo1 | Karyopherin subunit beta 1 / importin 1 |
| Kpnβ2/Ipoβ2/Tnpo1 | Karyopherin beta 2 / importin beta 2 / transportin 1 |
| Kpnβ2b/Ipo3/Tnpo2 | Karyopherin beta 2b / importin 3 / transportin 2 |
| Ipo4 | Importin 4 |
| Kpnβ3/Ipo5 | Karyopherin subunit beta 3 / importin 5 |
| Ipo7 | Importin 7 |
| Ipo8 | Importin 8 |
| Ipo9 | Importin 9 |
| Ipo11 | Importin 11 |
| Ipo12/Tnpo3 | Importin 12 / transportin 3 |

| **Adapter proteins** | |
|---|---|
| Kpnα1/Ipoα5 | Karyopherin subunit alpha 1 / importin alpha 5 |
| Kpnα2/Ipoα1 | Karyopherin subunit alpha 2 / importin alpha 1 |
| Kpnα3/Ipoα4 | Karyopherin subunit alpha 3 / importin alpha 4 |
| Kpnα4/Ipoα3 | Karyopherin subunit alpha 4 / importin alpha 3 |
| Kpnα5/Ipoα6 | Karyopherin subunit alpha 5 / importin alpha 6 |
| Kpnα6/Ipoα7 | Karyopherin subunit alpha 6 / importin alpha 7 |
| Kpnα7/Ipoα8 | Karyopherin subunit alpha 7 / importin alpha 8 |

| **Exportins** | |
|---|---|
| Crm1/Xpo1 | Chromosome region maintenance 1 / exportin 1 |
| CAS/Cse1L/Xpo2 | Cellular apoptosis susceptibility protein / chromosome segregation 1-like protein / exportin 2 |
| Xpo5 | Exportin 5 |
| Xpo6 | Exportin 6 |
| Xpo7 | Exportin 7 |
| XpoT | Exportin for transfer RNA |
| RanBP17 | Ran binding protein 17 |

| **Bidirectional** | |
|---|---|
| Kpn13/Ipo13 | Karyopherin 13 / importin 13 |
| Xpo4 | Exportin 4 |

| **Ran proteins** | |
|---|---|
| Ran | Ras related nuclear protein |
| RanBP1 | Ran binding protein 1 |
| RanBP6 | Ran binding protein 6 |
| RanGAP1 | Ran GTPase activating protein 1 |

Exosomes were next isolated from the conditioned media of HeLa, CaSki, WHCO5 and KYSE30 cells and mass spectrometry performed to determine whether the nuclear transport proteins might be present in the exosome fraction. Assessment of the integrity of the exosomes was first performed by TEM, and results revealed a size distribution of approximately 30 to 150 nm, consistent with the expected size of exosomes, as well as a mostly cup-shaped morphology, characteristic of exosomes (Figure 2). Mass spectrometry analysis using these isolated exosomes identified a total of 477, 737, 948 and 778 protein groups in all 3 replicates for CaSki, HeLa, WHCO5 and KYSE30, respectively. Results revealed a 40-50 % similarity in overall protein identities amongst cell lines and strong Pearson correlation coefficients were obtained when triplicate samples of the same cell line were compared to each other (on average > 0.9, data not shown). Notably, many of the highest ranked proteins identified by mass spectrometry (in terms of abundance) were typical exosome protein markers, providing confidence in the integrity of the exosome fractions. Furthermore, in comparing the exosome protein group lists obtained from HeLa, CaSki, WHCO5 and KYSE30 cells with those listed from other exosome studies in the extracellular vesicles database, Vesiclepedia, a high overlap in protein identities (> 63 %) was observed. Gene ontology enrichment analysis was performed, and cellular component ontology results revealed the highest fold enrichment in each cell line to be exosomes (where there was on average a 4.3-fold increase against the background database).

Interestingly, present amongst the listed proteins in the exosome fraction of each of the cancer cell lines were ten nuclear transport proteins: KPNβ1, KPNα2, IPO5, IPO7, TNPO1, CRM1, CAS, XPO7, RAN and RANGAP1 (Figure 3). CAS, KPNβ1 and RAN were the most abundant of the nuclear transport proteins present in the exosomes isolated from HeLa, CaSki, WHCO5 and KYSE30 cancer cells.

### Nuclear transport proteins are secreted at elevated levels by transformed and cancer cell lines compared to non-transformed epithelial cells

Having established by mass spectrometry that nuclear transport proteins were present in the secretome and exosome fraction of cervical and oesophageal cancer cells, protein levels were independently examined in the secretome of these cell lines by Western blot analysis and compared to that of a normal epithelial cell line, hTERT-RPE-1, as well as transformed cells, SVWI38. This was to determine whether nuclear transport proteins are secreted at elevated levels by cancer and transformed cells compared to normal. Western blot analysis was performed to evaluate the levels of KPNβ1, IPO5, IPO7, TNPO1, CRM1, CAS, KPNα2 and RAN in these cell lines. The results showed little to no detection of the 8 nuclear transport proteins in the secretome of hTERT-RPE1 cells with, in general, significantly higher levels observed in the transformed, cervical cancer and oesophageal cancer cell lines (Figure 4). The quantification of experiments performed in quadruplicate is shown in Figure 5. β-tubulin, which is not secreted from cells, was included to eliminate intracellular protein contamination as a confounding factor, while the Coomassie stained gel showed the protein profile of the secretome from all cell lines. Levels of Vimentin, a non-member of the nuclear transport protein family, were analysed, to confirm that not all secreted proteins were present at elevated levels in the transformed and cancer cells, and results showed decreased levels of secreted Vimentin in cancer cells, compared to normal (Figure 4, B). These findings suggest that the detection of nuclear transport proteins in the extracellular environment of cells associates with the transformed and cancer state.

In order to determine whether other cancer types similarly secrete high levels of nuclear transport proteins, protein levels were investigated in the secretome of breast cancer, MCF-7 and MDA-MB-231, and liver cancer, HepaRG, cells. Western blot analysis showed that all nuclear transport proteins were secreted at high levels by breast cancer and liver cancer cells, similar to levels secreted by cervical cancer and oesophageal cancer cells, and greater than levels secreted by non-cancer hTERT-RPE-1 cells (Figure 6). Interestingly, the highly aggressive and invasive triple negative breast cancer cells, MDA-MB-231, secreted substantially higher levels of nuclear transport proteins compared to the non-invasive luminal A subtype breast cancer cells, MCF-7, suggesting that nuclear transport protein secretion might be associated with cancer aggressiveness/metastatic potential. These results reveal that the secretion of nuclear transport proteins occurs across diverse cancer types and is not specific to cervical cancer and oesophageal cancer.

### Serum levels of KPNβ1, CRM1, KPNα2, CAS, RAN, IPO5 and TNPO1 are elevated in cervical/oesophageal cancer patients

Having established that multiple members of the nuclear transport protein family are secreted at elevated levels by cancer cells, the presence of these proteins was next determined in human serum samples. KPNβ1, CRM1, KPNα2, CAS, RAN, IPO5 and TNPO1 ELISA assays were performed, wherein a total of 220 serum samples were analysed: 73 sera from non-cancer subjects as controls; 74 sera from cervical cancer patients; and 73 sera from oesophageal cancer patients.

ELISA results showed that the serum levels of Kpnβ1, CRM1, CAS, IPO5 and TNPO1 were significantly elevated in cervical cancer patients compared to non-cancer controls, whereas serum KPNα2 and RAN levels were unchanged (Figure 7). Serum KPNβ1, CRM1, KPNα2, CAS, RAN, IPO5 and TNPO1 were each significantly higher in oesophageal cancer patients compared to non-cancer controls (Figure 8). The mean, median and range concentrations of serum KPNβ1, CRM1, KPNα2, CAS, RAN, IPO5 and TNPO1 in the non-cancer control subjects, cervical cancer and oesophageal cancer patients are shown in Table 2. Interestingly, KPNβ1, CAS and IPO5 were the most abundant of the seven nuclear transport proteins tested in patient serum samples, consistent with their high abundance observed in conditioned media *in vitro.* Overall, these results suggest that KPNβ1, CRM1, CAS, IPO5 and TNPO1 are potential biomarkers for the detection of cervical cancer, and that KPNβ1, CRM1, KPNα2, CAS, RAN, IPO5 and TNPO1 have potential as biomarkers for oesophageal cancer.

**Table 2. The mean, median and range concentrations of serum Kpnβ1, CRM1, Kpnα2, CAS, Ran, Ipo5 and Tnpo1 in the non-cancer subjects and cancer patients**

| | **Mean±SD** | | | | | | | **Median (range)** | | | | | | |
|---|---|---|---|---|---|---|---|---|---|---|---|---|---|---|
| | Kpnβ1 ng/ml | CRM1 pg/ml | Kpnα2 pg/ml | CAS ng/ml | Ran pg/ml | Ipo5 ng/ml | Tnpo1 pg/ml | Kpnβ1 ng/ml | CRM1 pg/ml | Kpnα2 pg/ml | CAS ng/ml | Ran pg/ml | Ipo5 ng/ml | Tnpo1 pg /ml |
| **Control (female only)** | 16.1±10 | 451±505 | 236±304 | 0.8±1.9 | 267±389 | 0.8±2.4 | 45.5±99 | 14.1 (0-57) | 214.3 (0-1469) | 126.5 (0-1319) | 0 (0-8.6) | 86 (0-1959 | 0.2 (0-17.5) | 6.7 (0-326.7) |
| **Cervical cancer** | 33.0±22 | 1082±1433 | 232±304 | 11.7±13 | 264±302 | 11.0±13 | 1595±1851 | 22.6 (8.5-85) | 483.7 (0-5504) | 268.1 (6-1434) | 6.2 (0-61) | 114 (0-1129) | 7.2 (0-66.7) | 797 (0-7047) |
| **Control (all)** | 16.5±10 | 385±488 | 198±276 | 0.9±2 | 206±353 | 0.9±2.3 | 63.8±207.9 | 14.3 (0-57) | 136.2 (0-1538) | 126.5 (0-1320) | 0 (0-14) | ( 61.3 (0-1969) | 0.2 (0-17.5) | 10 (0-1593) |
| **Oesophageal cancer** | 22.5±15 | 1177±1147 | 456±405 | 10.0±12 | 465±120 1 | 10.0±14 | 1555.4±1711 | 15.3 (5.8-82) | 817.8 (0-4760) | 268.1 (6-1435) | 5.4 (0-61) | 187.1 (0-9041) | 4.7 (0-70) | 810 (0-7447) |

As an ideal biomarker would allow for the detection of early stage cancer, serum KPNβ1, CRM1, KPNα2, CAS, RAN, IPO5 and TNPO1 levels were next compared in early-stage and late-stage cancer cases to the non-cancer control subjects. Results showed that serum KPNβ1, CRM1, CAS, IPO5 and TNPO1 were significantly elevated in early-stage cervical cancer cases (stages 1 and II) compared to the non-cancer controls (Figure 9), as well as in the late stage samples. This suggests that the upregulation and increased secretion of nuclear transporters, KPNβ1, CRM1, CAS, IPO5 and TNPO1 occurs in the early stages of cervical cancer progression, implicating them as potential early markers of the disease. The stage information for approximately half of the oesophageal cancer cases was not available with only 1 case recorded as early-stage (stages I/II combined). This coincides with the late detection of oesophageal cancer and thus limited the number of early-stage samples. As a result, the early-stage and late-stage oesophageal cancer cases could not be compared.

### Diagnostic value of KPNβ1, CRM1, KPNα2, CAS, RAN, IPO5 and TNPO1 as potential serum biomarkers of cervical and oesophageal cancers

To investigate the use of KPNβ1, CRM1, KPNα2, CAS, RAN, IPO5 and TNPO1 as potential biomarkers, a logistics regression analysis was performed, by plotting receiver operating characteristics (ROC) curves to analyse the ability of KPNβ1, CRM1, KPNα2, CAS, RAN, IPO5, TNPO1, and their combination, to discriminate cervical cancer and oesophageal cancer cases from non-cancer controls (Figures 10 and 11).

Results showed that IPO5 and TNPO1 were the best performing individual candidate biomarkers in discriminating between cervical cancer cases and non-cancer controls. They had the highest individual area under the ROC curve (AUC) values of 0.897 (Table 3). Importantly, all proteins had an AUC of greater than 0.5.

A combination of the seven candidate biomarkers gave the highest AUC of 0.944, suggesting excellent discriminating ability. Youden's index was calculated for each protein, and for the combination of all seven proteins. This is a score which maximises both sensitivity and specificity and gives an indication of the maximum effectiveness of a biomarker. The combination of all 7 biomarkers yielded the highest Youden's index of 0.793, based on a sensitivity of 92.5 % at 86.8 % specificity (Table 3).

Kpnα2, Ran and Ipo5 was the best 3-protein combination, giving an AUC of 0.928 for cervical cancer. Ran and Ipo5, with an AUC value of 0.921, was the best performing 2-pair combination, although the combination of Kpnβ1 and Ipo5 was also good, with an AUC value of 0.906.

**Table 3: Classification performance of the candidate biomarkers in the cervical cancer cases**

| **Candidate biomarkers** | **AUC±SE (95% Confidence interval)** | ***p*-value for AUC** | **Youden's index** | **Sensitivity** | **Specificity** |
|---|---|---|---|---|---|
| **Kpnβ1 (ng/ml)** | 0.77±0.04 (0.66 - 0.85) | <0.0001 | 0.366 | 90.7 | 45.9 |
| **CRM1 (pg/ml)** | 0.64±0.05 (0.54 - 0.74) | <0.01 | 0.257 | 35.2 | 90.5 |
| **Kpnα2 (pg/ml)** | 0.51±0.05 (0.41 - 0.61) | 0.82 | 0.14 | 66.7 | 47.3 |
| **CAS (ng/ml)** | 0.85±0.03 (0.78 - 0.91) | <0.0001 | 0.612 | 81.5 | 79.7 |
| **Ran (pg/ml)** | 0.52±0.05 (0.41 - 0.61) | 0.82 | 0.102 | 42.6 | 67.6 |
| **Ipo5 (ng/ml)** | 0.897±0.03 (0.83 - 0.94) | <0.0001 | 0.74 | 88.9 | 85.1 |
| **Tnpo1 (pg/ml)** | 0.897±0.03 (0.84 - 0.96) | <0.0001 | 0.73 | 90.6 | 82.4 |
| **Combination** | 0.944 | <0.0001 | 0.793 | 92.5 | 86.8 |

Similarly, in discriminating oesophageal cancer cases from the non-cancer controls, IPO5 was the best performing individual biomarker, with an AUC of 0.91. Again, the highest diagnostic capacity was achieved when the seven candidate biomarkers were combined, with an AUC of 0.963 and a Youden's index of 0.828 based on 95.3 % sensitivity at 87.5 % specificity (Figure 11, Table 4).

Kpnα2, Ran and Ipo5 appear to be the best 3-protein combination, giving an AUC of 0.939 for oesophageal cancer. Ran and Ipo5, with an AUC value of 0.932 for oesophageal cancer, was the best performing 2-pair combination, followed by the combination of Kpnβ1 and Ipo5, with an AUC value of 0.932. Kpnβ1 performed poorly.

**Table 4: Classification performance of the candidate biomarkers in the oesophageal cancer cases**

| **Candidate biomarkers** | **AUC±SE (95% Confidence interval)** | **1 *p*-value for AUC** | **Youden's index** | **Sensitivity** | **Specificity** |
|---|---|---|---|---|---|
| **Kpnβ1 (ng/ml)** | 0.62±0.05 (0.53 - 0.72) | <0.01 | 0.268 | 31.8 | 95 |
| **CRM1 (pg/ml)** | 0.75±0.04 (0.67 - 0.83) | <0.0001 | 0.409 | 57.6 | 83.3 |
| **Kpnα2 (pg/ml)** | 0.73±0.04 (0.65 - 0.81) | <0.0001 | 0.394 | 72.7 | 66.7 |
| **CAS (ng/ml)** | 0.86+0.03 (0.79 - 0.92) | <0.0001 | 0.638 | 78.8 | 85 |
| **Ran (pg/ml)** | 0.63±0.05 (0.53 - 0.73) | <0.05 | 0.299 | 68.2 | 61.7 |
| **Ipo5 (ng/ml)** | 0.91+0.03 (0.85 - 0.95) | <0.0001 | 0.696 | 87.9 | 81.7 |
| **Tnpo1 (pg/ml)** | 0.875+0.04 (0.80 - 0.95) | <0.0001 | 0.743 | 92.2 | 82.1 |
| **Combination** | 0.963 | <0.0001 | 0.828 | 95.3 | 87.5 |

It was next determined whether the levels of any nuclear transport protein, or combination of family members, would be able to distinguish between cervical and oesophageal cancer cases. Interestingly, KPNβ1 was present at significantly reduced levels in oesophageal cancer serum samples, compared to cervical cancer serum samples, while KPNα2 was present at significantly increased levels in oesophageal cancer serum samples, compared to cervical cancer serum samples (Figure 12). None of the other biomarkers displayed significantly different serum levels between cervical cancer and oesophageal cancer samples. A ROC curve analysis was performed, which showed that the combination of KPNβ1 and KPNα2 was able to distinguish between cervical cancer and oesophageal cancer cases with an AUC of 0.787 and a sensitivity of 77.9 % at 69.6 % specificity. While this is considered acceptable discriminating ability and suggests that the levels of certain nuclear transport proteins might hold potential in discriminating between cancer types, the most significant findings in this study are the superior AUC values obtained when all biomarkers are combined and used to discriminate cancer cases from non-cancer controls.

These findings indicate that a diagnostic test, measuring levels of the nuclear transport proteins, KPNβ1, CRM1, KPNα2, CAS, RAN, IPO5 and TNPO1, in patient serum, has the potential to discriminate cancer patients from healthy controls.

### DISCUSSION

In this study, the potential of nuclear transport proteins as cervical and oesophageal cancer diagnostic biomarkers was investigated. The Applicant has shown that several of these protein family members are released from cancer cells in exosomes, and that the levels of most of the nuclear transport proteins investigated are significantly elevated in cervical and oesophageal cancer patient serum compared to that of healthy controls.

Blood serum was chosen as the source of secreted protein biomarkers in this study because it encounters virtually all cells in the body during its circulation, thereby accruing a vast number of secreted proteins. The concentration of KPNβ1, CRM1, KPNα2, CAS, RAN, IPO5 and TNPO1 in serum samples from a cohort of non-cancer subjects, cervical cancer and oesophageal cancer patients was measured, and amongst the seven nuclear transport proteins investigated, KPNβ1, CRM1, CAS, IPO5 and TNPO1 were found to be present at significantly elevated levels in cervical cancer patient serum compared to that of the non-cancer control subjects. It is worth noting that even early stage cervical cancer patients had significantly increased levels of KPNβ1, CRM1, CAS, IPO5 and TNPO1 in their blood compared to healthy controls, suggesting that these proteins may be useful biomarkers for the early detection of cervical cancer. The diagnosis of a malignancy in its early stages greatly increases the chances of successful treatment and improved patient outcome. Interestingly, all seven candidate biomarkers were significantly elevated in oesophageal cancer patient serum compared to that of the non-cancer control subjects.

One of the qualities of an ideal biomarker is the ability of the biomarker to minimise false-positive and false-negative values at diagnosis. A logistics regression analysis was therefore performed by plotting ROC curves and calculating the AUC to determine the sensitivity and specificity of the candidate biomarkers and their ability to discriminate between cancer cases and non-cancer controls. The AUC is a single index for measuring the performance of a diagnostic test, and the closer the AUC is to 1, the better the overall performance of the diagnostic test to correctly identify diseased and non-diseased subjects. In this study, IPO5 and TNPO1 were the best performing individual candidate biomarkers in discriminating between cervical cancer cases and non-cancer controls. They had the highest individual AUC values (0.897), superior to KPNβ1, CRM1, KPNα2, CAS and RAN. A combination of the seven candidate biomarkers gave the highest AUC of 0.944 with a sensitivity of 92.5 % at 86.8 % specificity.

In discriminating oesophageal cancer cases from the non-cancer controls, IPO5 (AUC=0.91 with 87.9 % sensitivity at 81.7 % specificity) performed best, and the highest diagnostic capacity was achieved when the seven candidate biomarkers were combined, with an AUC of 0.963 and 95.3 % sensitivity at 87.5 % specificity. This study showed that IPO5 outperformed KPNα2 as a potential biomarker for oesophageal cancer, and had a further improved performance when combined with KPNβ1, CRM1, KPNα2, CAS, RAN and TNPO1.

To the best of the Applicant's knowledge, this study is the first to show by mass spectrometry and antibody-based techniques that multiple nuclear transport proteins are present in the secretome (and exosomes) of cervical and oesophageal cancer cells. Similarly, the results show that nuclear transport proteins are secreted by breast cancer and liver cancer cells, suggesting that this could be a feature of cancer cells in general. Additionally, this study is the first to investigate serum KPNβ1, CRM1, KPNα2, CAS, RAN, IPO5 and TNPO1 as a panel of biomarkers for the diagnosis of cervical and oesophageal cancers. Taken together, these findings show that measuring the levels of KPNβ1, CRM1, KPNα2, CAS, RAN, IPO5 and TNPO1 (especially as a panel of biomarkers) in blood could serve as a diagnostic tool for the detection of cervical cancer, oesophageal cancer and a wide range of other cancer types too.

## Claims

1. A method of diagnosing cancer in a subject, the method comprising the step of testing a blood sample from a subject for the presence of Ipo5 (Importin 5) and at least one other biomarker, wherein the at least one other biomarker is selected from the group consisting of Ran (Ras-related nuclear protein) and Kpnβ1 (Karyopherin beta 1).

2. A method according to claim 1, which comprises testing the sample for the presence of:
at least Ipo5 and Ran;
at least Ipo5 and Kpnβ1; or
at least Ipo5, Ran and Kpnβ1.

3. A method according to claim 1, which comprises additionally testing the sample for at least one other biomarker selected from the group consisting of Kpnα2 (Karyopherin alpha 2), CRM1 (XP01 / Chromosome Region Maintenance 1 Protein Homolog), CAS (Cellular apoptosis-susceptibility protein) and Transportin 1.

4. A method according to claim 1, which comprises testing the sample for the presence of at least 5 biomarkers selected from the group consisting of: Ipo5, Ran, Kpnβ1, Kpnα2, CRM1, CAS and Transportin 1.

5. A method according to claim 1, which comprises testing the sample for the presence of at least 6 biomarkers selected from the group consisting of Ipo5, Ran, Kpnβ1, Kpnα2, CRM1, CAS and Transportin 1.

6. A method according to claim 1, which comprises testing the sample for the presence of the following biomarkers: Ipo5, Ran, Kpnβ1, Kpnα2, CRM1, CAS and Transportin 1.

7. A method according to any one of claims 1 to 6, which does not diagnose a specific type of cancer.

8. A method according to any one of claims 1 to 6, which detects at least cervical cancer, oesophageal cancer, liver and breast cancer, if any of these cancers are present in the subject.

9. A method according to any one of claims 1 to 6, wherein the cancer is cervical cancer.

10. A method according to any one of claims 1 to 6, wherein the cancer is oesophageal cancer.

11. A method according to any one of claims 1 to 6, wherein the cancer is breast cancer.

12. A method according to any one of claims 1 to 6, wherein the cancer is liver cancer.

13. A point-of-care device for diagnosing cancer, the device comprising:
a means for receiving a blood sample from a subject suspected of having cancer;
capture agents for binding Ipo5 and at least one other biomarker, wherein the at least one other biomarker is selected from the group consisting of Ran and Kpnβ1; and
at least one indicator which indicates when the capture agents bind to the biomarkers.

14. A kit for diagnosing cancer, the kit comprising the following:
capture agents for binding Ipo5 and at least one other biomarker, wherein the at least one other biomarker is selected from the group consisting of Ran and Kpnβ1;
means for obtaining or receiving a blood sample from a subject;
a point-of-care device for diagnosing cancer according to claim 13; and
instructions, in electronic or paper form, for performing the method as claimed in any one of claims 1 to 12 and diagnosing cancer.

15. A computer implemented method for diagnosing cancer in a subject, the computer performing steps comprising:
receiving inputted subject data comprising values for levels of Ipo5 and at least one other biomarker, wherein the at least one other biomarker is selected from the group consisting of Ran and Kpnβ1, in a blood sample from the subject;
comparing these values with predetermined values for the biomarkers;
determining whether the subject has cancer; and
displaying information regarding the diagnosis of the subject.

## Patentansprüche

1. Verfahren zum Diagnostizieren von Krebs in einem Subjekt, wobei das Verfahren den Schritt eines Testens einer Blutprobe von einem Subjekt auf das Vorhandensein von Ipo5 (Importin 5) und mindestens einem anderen Biomarker umfasst, wobei der mindestens eine andere Biomarker aus der Gruppe ausgewählt ist, die aus Ran (Ras-verwandtes nukleäres Protein) und Kpnβ1 (Karyopherin beta 1) besteht.

2. Verfahren nach Anspruch 1, das Testen der Probe auf das Vorhandensein von Folgendem umfasst:
mindestens Ipo5 und Ran;
mindestens Ipo5 und Kpnβ1; oder
mindestens Ipo5, Ran und Kpnβ1.

3. Verfahren nach Anspruch 1, das zusätzlich Testen der Probe auf mindestens einen anderen Biomarker umfasst, der aus der Gruppe ausgewählt ist, die aus Kpnα2 (Karyopherin alpha 2), CRM1 (XP01 / Chromosome Region Maintenance 1 Protein Homolog), CAS (Cellular Apoptosis Susceptibility Protein) und Transportin 1 besteht.

4. Verfahren nach Anspruch 1, das Testen der Probe auf das Vorhandensein von mindestens 5 Biomarkern umfasst, die aus der Gruppe ausgewählt sind, die aus Folgenden besteht: Ipo5, Ran, Kpnβ1, Kpnα2, CRM1, CAS und Transportin 1.

5. Verfahren nach Anspruch 1, das Testen der Probe auf das Vorhandensein von mindestens 6 Biomarkern umfasst, die aus der Gruppe ausgewählt sind, die aus Ipo5, Ran, Kpnβ1, Kpnα2, CRM 1, CAS und Transportin 1 besteht.

6. Verfahren nach Anspruch 1, das Testen der Probe auf das Vorhandensein der folgenden Biomarker umfasst: Ipo5, Ran, Kpnβ1, Kpnα2, CRM1, CAS und Transportin 1.

7. Verfahren nach einem der Ansprüche 1 bis 6, das keine spezifische Krebsart diagnostiziert.

8. Verfahren nach einem der Ansprüche 1 bis 6, das mindestens Gebärmutterhalskrebs, Speiseröhrenkrebs, Leber- und Brustkrebs nachweist, wenn eine beliebige dieser Krebsarten bei dem Subjekt vorliegt.

9. Verfahren nach einem der Ansprüche 1 bis 6, wobei der Krebs Gebärmutterhalskrebs ist.

10. Verfahren nach einem der Ansprüche 1 bis 6, wobei der Krebs Speiseröhrenkrebs ist.

11. Verfahren nach einem der Ansprüche 1 bis 6, wobei der Krebs Brustkrebs ist.

12. Verfahren nach einem der Ansprüche 1 bis 6, wobei der Krebs Leberkrebs ist.

13. Point-of-Care-Vorrichtung zum Diagnostizieren von Krebs, wobei die Vorrichtung Folgendes umfasst:
ein Mittel zum Empfangen einer Blutprobe von einem Subjekt, bei dem der Verdacht auf Krebs besteht;
Einfangmittel zum Binden von Ipo5 und mindestens einem anderen Biomarker, wobei der mindestens eine andere Biomarker aus der Gruppe ausgewählt ist, die aus Ran und Kpnβ1 besteht; und
mindestens einen Indikator, der angibt, wann die Einfangmittel an die Biomarker binden.

14. Kit zum Diagnostizieren von Krebs, wobei das Kit Folgendes umfasst:
Einfangmittel zum Binden von Ipo5 und mindestens einem anderen Biomarker, wobei der mindestens eine andere Biomarker aus der Gruppe ausgewählt ist, die aus Ran und Kpnβ1 besteht;
Mittel zum Erhalten oder Empfangen einer Blutprobe von einem Subjekt;
eine Point-of-Care-Vorrichtung zum Diagnostizieren von Krebs nach Anspruch 13; und
Anweisungen in elektronischer Form oder Papierform zum Durchführen des Verfahrens nach einem der Ansprüche 1 bis 12 und zum Diagnostizieren von Krebs.

15. Computerimplementiertes Verfahren zum Diagnostizieren von Krebs bei einem Subjekt, wobei der Computer Schritte ausführt, die Folgendes umfassen:
Empfangen von eingegebenen Subjektdaten, die Werte für die Spiegel von Ipo5 und mindestens einem anderen Biomarker umfassen, wobei der mindestens eine andere Biomarker aus der Gruppe ausgewählt ist, die aus Ran und Kpnβ1 besteht, in einer Blutprobe des Subjekts;
Vergleichen dieser Werte mit vorbestimmten Werten für die Biomarker;
Bestimmen, ob das Subjekt Krebs aufweist; und
Anzeigen von Informationen bezüglich der Diagnose des Subjekts.

## Revendications

1. Procédé de diagnostic de cancer chez un sujet, le procédé comprenant l'étape consistant à tester un échantillon sanguin d'un sujet pour la présence d'Ipo5 (Importine 5) et d'au moins un autre biomarqueur, ledit au moins un autre biomarqueur étant choisi dans le groupe constitué de Ran (protéine nucléaire apparentée à Ras) et de Kpnβ1 (karyopherine bêta 1).

2. Procédé selon la revendication **1,** qui comprend le test de l'échantillon pour la présence:
d'au moins Ipo5 et Ran ;
d'au moins Ipo5 et Kpnβ1 ; ou
d'au moins Ipo5, Ran et Kpnβ1.

3. Procédé selon la revendication 1, qui comprend en outre le test de l'échantillon pour au moins un autre biomarqueur choisi dans le groupe constitué par Kpnα2 (karyophérine alpha 2), CRM1 (XP01/homologue de la protéine de maintenance de la région chromosomique 1), CAS (protéine de susceptibilité à l'apoptose cellulaire) et Transportin 1.

4. Procédé selon la revendication 1, qui comprend le test de l'échantillon pour la présence d'au moins 5 biomarqueurs choisis dans le groupe constitué par : Ipo5, Ran, Kpnβ1, Kpnα2, CRM1, CAS et Transportin 1.

5. Procédé selon la revendication 1, qui comprend le test de l'échantillon pour la présence d'au moins 6 biomarqueurs choisis dans le groupe constitué par Ipo5, Ran, Kpnβ1, Kpnα2, CRM1, CAS et Transportin 1.

6. Procédé selon la revendication 1, qui comprend le test de l'échantillon pour la présence des biomarqueurs suivants : Ipo5, Ran, Kpnβ1, Kpnα2, CRM1, CAS et Transportin 1.

7. Procédé selon l'une quelconque des revendications 1 à 6, qui ne permet pas de diagnostiquer un type spécifique de cancer.

8. Procédé selon l'une quelconque des revendications 1 à 6, qui détecte au moins le cancer du col de l'utérus, le cancer de l'œsophage, le cancer du foie et le cancer du sein, si l'un de ces cancers est présent chez le sujet.

9. Procédé selon l'une quelconque des revendications 1 à 6, dans lequel le cancer est le cancer du col de l'utérus.

10. Procédé selon l'une quelconque des revendications 1 à 6, dans lequel le cancer est le cancer de l'œsophage.

11. Procédé selon l'une quelconque des revendications 1 à 6, dans lequel le cancer est le cancer du sein.

12. Procédé selon l'une quelconque des revendications 1 à 6, dans lequel le cancer est le cancer du foie.

13. Dispositif de diagnostic de cancer au point d'intervention, le dispositif comprenant :
un moyen de réception d'un échantillon sanguin d'un sujet suspecté d'avoir un cancer ;
des agents de capture pour lier Ipo5 et au moins un autre biomarqueur, l'au moins un autre biomarqueur étant choisi dans le groupe constitué par Ran et Kpnβ1 ; et
au moins un indicateur qui indique quand les agents de capture se lient aux biomarqueurs.

14. Kit de diagnostic de cancer, le kit comprenant les éléments suivants :
des agents de capture pour lier Ipo5 et au moins un autre biomarqueur, l'au moins un autre biomarqueur étant choisi dans le groupe constitué par Ran et Kpnβ1 ;
un moyen pour obtenir ou recevoir un échantillon sanguin d'un sujet ;
un dispositif de diagnostic de cancer au point d'intervention selon la revendication 13 ; et
des instructions, sous forme électronique ou papier, pour mettre en œuvre le procédé selon l'une quelconque des revendications 1 à 12 et diagnostiquer le cancer.

15. Procédé mis en œuvre par ordinateur pour diagnostiquer un cancer chez un sujet, l'ordinateur effectuant des étapes comprenant :
la réception de données de sujet saisies comprenant des valeurs pour des niveaux d'Ipo5 et d'au moins un autre biomarqueur, ledit au moins un autre biomarqueur étant sélectionné dans le groupe constitué de Ran et Kpnβ1, dans un échantillon de sang du sujet ;
la comparaison de ces valeurs avec des valeurs prédéterminées pour les biomarqueurs ;
la détermination si le sujet a un cancer ; et
l'affichage d'informations concernant le diagnostic du sujet.
